# EUROPÄISCHE PATENTANMELDUNG

(11) **EP 4 813 361 A1**
(43) Veröffentlichungstag der Anmeldung: **30.09.2026**
(21) Anmeldenummer: 25166496.7
(22) Anmeldetag: 26.03.2025
(51) Int. Cl.: A61F 11/08, A61F 11/10, A61F 2/00

(54) **OHRSTÖPSELEINFÜHRVORRICHTUNG**

(71) Anmelder: Thumm, Christian, 84453 Mühldorf (DE)
(72) Erfinder: Thumm, Christian, 84453 Mühldorf (DE)
(74) Vertreter: Gössmann, Christoph Tassilo

(57) **Zusammenfassung**

Ohrstöpseleinführvorrichtung (1) die ein Magazin aufweist, das zur Aufnahme für kompressible und expandible Ohrstöpsel aus Schaumstoff geeignet ist sowie eine Komprimiervorrichtung, in der ein kompressibler und expandibler Ohrstöpsel der Länge nach komprimierbar ist sowie eine Ohrstöpselfördervorrichtung (3), um den Ohrstöpsel aus dem Magazin in die Komprimiervorrichtung und dann in den Ohrgang einzuführen.

## Beschreibung

Die Erfindung betrifft eine Ohrstöpseleinführvorrichtung.

Offenporige kompressible und expandible Ohrstöpsel aus Schaumstoff sind oft schwierig einzuführen, insbesondere, wenn der Einführende kalte, durch Vibrationen gefühllose Finger oder schmutzige Hände hat, da diese Ohrstöpsel vor dem Einführen zusammen gedrückt werden müssen, damit sie in den Ohrgang passen und dort wieder expandieren können.

Es ist Aufgabe der Erfindung den Stand der Technik zu verbessern und insbesondere eine Vorrichtung zur Verfügung zu stellen, die es ermöglicht einfach und sauber offenporige kompressible und expandible Ohrstöpsel aus Schaumstoff sicher in einen Ohrgang aus einem Magazin einzuführen.

Die Aufgabe wird durch die Ansprüche gelöst.

Ohrstöpseleinführvorrichtung, die ein Magazin aufweist, das zur Aufnahme für vorzugsweise offenporige, kompressible und expandible Ohrstöpsel aus Schaumstoff geeignet ist sowie eine Komprimiervorrichtung, in der ein kompressibler und expandibler Ohrstöpsel der Länge nach komprimierbar ist sowie eine Ohrstöpselfördervorrichtung, um den Ohrstöpsel aus dem Magazin in die Komprimiervorrichtung und dann in den Ohrgang einzuführen.

Die erfindungsgemäße Ohrstöpseleinführvorrichtung weist vorzugsweise ein Magazin auf, das vorzugsweise einen rotierbaren kreisförmigen Formkörper aufweist, der Bohrungen in der Tiefe und dem Durchmesser von zumindest der Länge und dem Durchmesser eines Ohrstöpsels aufweist, wobei die Ohrstöpsel in dem Magazin kreisförmig angeordnet sind.

Eine andere Ausführungsform des Magazins der erfindungsgemäßen Ohrstöpseleinführvorrichtung wäre ein Magazin, das ein quaderförmiger Formkörper ist, in dem die Ohrstöpsel übereinander angeordnet sind und über eine Fördervorrichtung von unten nach oben förderbar sind. Die Fördervorrichtung könnte eine Feder sein, die unter den Ohrstöpseln angeordnet ist und die Ohrstöpsel sukzessive nach oben drücken, wenn ein Ohrstöpsel entnommen wird. Eine andere Fördervorrichtung wäre ein Endlosförderer, der vorzugsweise elipsenförmig läuft.

Die erfindungsgemäße Ohrstöpseleinführvorrichtung weist eine Komprimiervorrichtung auf, die eine Ohrstöpselaufnahme aufweist, vorzugsweise eine nach oben offene Hülse, in der der Ohrstöpsel mit einem Einführkolben förderbar ist, der vorzugsweise an seinem Ende die Form einer konkaven Platte aufweist, mit dem er mit dem Ohrstöpsel formschlüssig in Berührung kommt. Dadurch erfolgt die Komprimierung des Ohrstöpsels.

Die erfindungsgemäße Ohrstöpseleinführvorrichtung kann auch als Komprimiervorrichtung eine nach oben und den Enden offene Hülse aufweisen, in der der Ohrstöpsel mit einem flexiblen Band, vorzugsweise aus Metall oder Teflon, das mit dem Ohrstöpsel formschlüssig in Berührung kommt und den Ohrstöpsel umschließt und durch Ziehen am Band den Ohrstöpsel komprimiert.

Die erfindungsgemäße Ohrstöpseleinführvorrichtung kann auch als Komprimiervorrichtung kreisförmig angeordnete Stifte, Stempel, Lamellen, Backen, Kolben, Zylinder aufweisen, die vorzugsweise gleichmäßig zur Mitte ausfahrbar sind, um so den Durchmesser der Ohrstöpselaufnahme der Komprimiervorrichtung zu verkleinern und einen dort in der Mitte angeordneten Ohrstöpsel, der dort eingeführt wurde, zu komprimieren.

Die erfindungsgemäße Ohrstöpseleinführvorrichtung kann auch als Komprimiervorrichtung eingehauste Ballone, Wülste aufweisen, die vorzugsweise gleichmäßig zur Mitte aufblasbar oder mit Flüssigkeit befüllbar sind, um so den Durchmesser der Ohrstöpselaufnahme der Komprimiervorrichtung zu verkleinern und einen dort in der Mitte angeordneten Ohrstöpsel, der dort eingeführt wurde, zu komprimieren.

Die Ohrstöpselfördervorrichtung weist vorzugsweise einen Förderkolben auf, der den Ohrstöpsel aus dem Magazin in die Komprimiervorrichtung und einen weiteren Kolben, den Einführkolben, der den komprimierten Ohrstöpsel von der Komprimiervorrichtung in den Ohrgang befördert.

Die erfindungsgemäße Einstellvorrichtung kann auch vorzugsweise eine Einstellvorrichtung aufweisen, die am Ausgang der Ohrstöpseleinführvorrichtung, also an der Stelle, an der der Ohrstöpsel in den Ohrgang eingeführt wird, eine Einstellvorrichtung aufweist, die vorzugsweise ein Schraubengewinde aufweist, um den Abstand zwischen dem Ausgang der Ohrstöpseleinführvorrichtung und der Einstellvorrichtung zum Ohrgang zu verändern, vorzugsweise zu verlängern, um so den Weg einzustellen, wie weit der Ohrstöpsel in den Ohrgang einführbar ist.

### Figuren

**Figur 1**

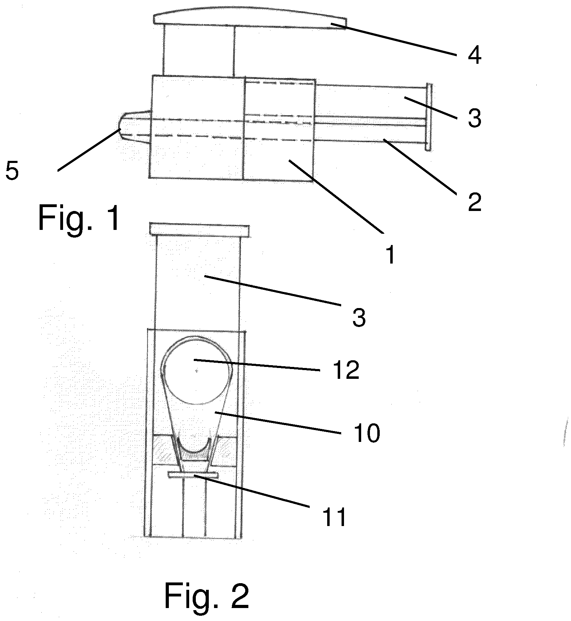

   Figur 1 zeigt die gesamte Ohrstöpseleinführvorrichtung 1 im seitlichen Querschnitt. Die Ohrstöpselfördervorrichtung 1 weist einen Förderkolben 3 mit Daumendrückplatte auf, der den Ohrstöpsel aus dem Magazin in die Komprimiervorrichtung und einen weiteren Kolben, den Einführkolben 2, der den komprimierten Ohrstöpsel von der Komprimiervorrichtung in den Ohrgang befördert, wobei mit dem Komprimierhebel 4, die Komprimiervorrichtung bedient wird.
   Des Weiteren ist eine Einstellvorrichtung 5 zu sehen, die am Ausgang der Ohrstöpseleinführvorrichtung, also an der Stelle, an der der Ohrstöpsel in den Ohrgang eingeführt wird, eine Einstellvorrichtung aufweist, die vorzugsweise ein Schraubengewinde aufweist, um den Abstand zwischen dem Ausgang der Ohrstöpseleinführvorrichtung und der Einstellvorrichtung zum Ohrgang zu verändern, vorzugsweise zu verlängern, um so den Weg einzustellen, wie weit der Ohrstöpsel in den Ohrgang einführbar ist.
**Figur 2**
   Figur 2 zeigt die Komprimiervorrichtung mit dem Förderkolben 3 der einen Ohrstöpsel 12 in die Komprimiervorrichtung in Form eines flexiblen Metallkomprimierbandes 10, das durch eine Metallklammer 11 zusammen gehalten wird und nach unten ziehbar ist, um den Ohrstöpsel 12 zu komprimieren.
**Figur 3**

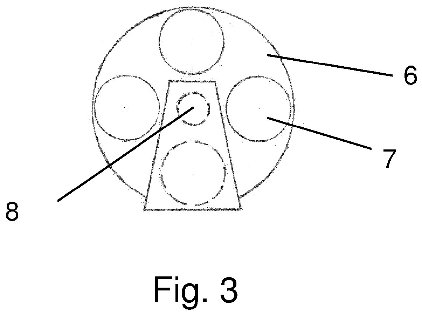

   Figur 3 zeigt einen rotierbaren kreisförmigen Formkörper 6, der Bohrungen 7 in der Tiefe und dem Durchmesser von zumindest der Länge und dem Durchmesser eines Ohrstöpsels aufweist, wobei die Ohrstöpsel in dem Magazin 6 kreisförmig angeordnet sind, wobei der kreisförmigen Formkörper 6, ein Rotationsmagazin 6 mit Ohrstöpselaufnahmeöffnungen 7, um eine Rotationsachse 8 rotiert.
**Figur 4**

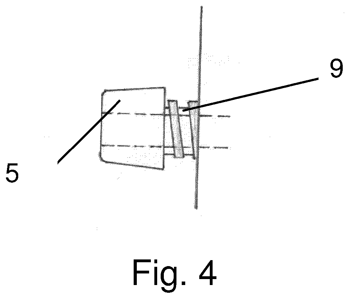

   Figur 4 zeigt eine Einstellvorrichtung 5, die ein Schraubengewinde 9 aufweist, um den Abstand zwischen dem Ausgang der Ohrstöpseleinführvorrichtung und der Einstellvorrichtung zum Ohrgang zu verändern, vorzugsweise zu verlängern, um so den Weg einzustellen, wie weit der Ohrstöpsel in den Ohrgang einführbar ist.

## Patentansprüche

1. Ohrstöpseleinführvorrichtung 1, **dadurch gekennzeichnet, dass** sie ein Magazin aufweist, das zur Aufnahme für kompressible und expandible Ohrstöpsel 12 aus Schaumstoff geeignet ist sowie eine Komprimiervorrichtung, in der ein kompressibler und expandibler Ohrstöpsel 12 der Länge nach komprimierbar ist sowie eine Ohrstöpselfördervorrichtung 1, um den Ohrstöpsel 12 aus dem Magazin in die Komprimiervorrichtung und dann in den Ohrgang einzuführen.

2. Ohrstöpseleinführvorrichtung nach Anspruch 1 **dadurch gekennzeichnet, dass** das Magazin einen rotierbaren kreisförmigen Formkörper 6 aufweist, der Bohrungen 7 in der Tiefe und dem Durchmesser von zumindest der Länge und dem Durchmesser eines Ohrstöpsels 12 aufweist, wobei die Ohrstöpsel 12 kreisförmig angeordnet sind.

3. Ohrstöpseleinführvorrichtung nach Anspruch 1 **dadurch gekennzeichnet, dass** das Magazin 6 ein quaderförmiger Formkörper ist, in dem die Ohrstöpsel 12 übereinander angeordnet sind, und über eine Fördervorrichtung von unten nach oben förderbar sind.

4. Ohrstöpseleinführvorrichtung nach Anspruch 1 **dadurch gekennzeichnet, dass** die Komprimiervorrichtung eine nach oben und den Enden offene Hülse aufweist, in der der Ohrstöpsel 12 mit einem Einführkolben 2 förderbar ist, der an seinem Ende die Form einer konkaven Platte aufweist, mit dem er mit dem Ohrstöpsel 12 formschlüssig in Berührung kommt, aufweist.

5. Ohrstöpseleinführvorrichtung nach Anspruch 1 **dadurch gekennzeichnet, dass** die Komprimiervorrichtung eine nach oben offene Hülse aufweist, in der der Ohrstöpsel 12 mit einem flexiblen Band 10, das mit dem Ohrstöpsel 12 formschlüssig in Berührung kommt und den Ohrstöpsel 12 umschließt.

6. Ohrstöpseleinführvorrichtung nach Anspruch 5, **dadurch gekennzeichnet, dass** das flexible Band 10 aus Metall oder Teflon ist.

7. Ohrstöpseleinführvorrichtung nach Anspruch 1 **dadurch gekennzeichnet, dass** die Komprimiervorrichtung als Komprimiervorrichtung kreisförmig angeordnete Stifte, Stempel, Lamellen, Backen, Kolben, Zylinder aufweist, die zur Mitte ausfahrbar sind, um so den Durchmesser der Ohrstöpselaufnahme der Komprimiervorrichtung zu verkleinern und einen dort in der Mitte angeordneten Ohrstöpsel, der dort eingeführt wurde, zu komprimieren.

8. Ohrstöpseleinführvorrichtung nach Anspruch 1 **dadurch gekennzeichnet, dass** die Komprimiervorrichtung eingehauste Ballone, Wülste aufweist, die zur Mitte aufblasbar oder mit Flüssigkeit befüllbar sind, um so den Durchmesser der Ohrstöpselaufnahme der Komprimiervorrichtung zu verkleinern und einen dort in der Mitte angeordneten Ohrstöpsel, der dort eingeführt wurde, zu komprimieren.

9. Ohrstöpseleinführvorrichtung nach einem oder mehreren der vorhergehenden Ansprüche, **dadurch gekennzeichnet, dass** die Ohrstöpselfördervorrichtung einen Förderkolben 3 aufweist, der den Ohrstöpsel aus dem Magazin in die Komprimiervorrichtung und einen weiteren Kolben, den Einführkolben 2, der den komprimierten Ohrstöpsel 12 von der Komprimiervorrichtung in den Ohrgang befördert.

10. Ohrstöpseleinführvorrichtung nach einem oder mehreren der vorhergehenden Ansprüche, **dadurch gekennzeichnet, dass** die Ohrstöpselfördervorrichtung eine Einstellvorrichtung 5 aufweist, die den Abstand zwischen dem Ausgang der Ohrstöpseleinführvorrichtung und der Einstellvorrichtung zum Ohrgang verändert, vorzugsweise zu verlängern, um so den Weg einzustellen, wie weit der Ohrstöpsel 12 in den Ohrgang einführbar ist.
